(19) Europäisches Patentamt
European Patent Office
Office européen des brevets

(11) Numéro de publication : **0 028 964**
**B1**

(12) **FASCICULE DE BREVET EUROPÉEN**

(45) Date de publication du fascicule du brevet : **15.12.82**

(51) Int. Cl.³ : **C 07 B 13/00**, C 07 C143/08, C 07 C139/00

(21) Numéro de dépôt : **80401533.7**

(22) Date de dépôt : **29.10.80**

(54) **Procédé de préparation de difluorométhane sulfonates alcalins.**

(30) Priorité : **13.11.79 FR 7927889**

(43) Date de publication de la demande : **20.05.81 (Bulletin 81/20)**

(45) Mention de la délivrance du brevet : **15.12.82 Bulletin 82/50**

(84) Etats contractants désignés : **AT BE CH DE FR GB IT LI NL SE**

(56) Documents cités :
**DE A 2 545 644**
**FR A 1 139 251**
**US A 3 992 432**
**JOURNAL OF THE CHEMICAL SOCIETY, 1960, part III, The Chemical Society Letchwork Herts GB W.V. FARRAR : « Arylamides of Halogenated Methane- and Ethanesulphonic Acids », pages 3 058-3 062**
**HOUBEN-WEYL. 4e Ed. Tome IX. 1955. p. 372.**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Langlois, Bernard**
**91, rue Du Guesclin**
**F-69006 - Lyon (FR)**

(74) Mandataire : **Cazes, Jean-Marie et al**
**RHONE-POULENC RECHERCHES Service Brevets Chimie et Polymères 25, quai Paul Doumer**
**F-92408 Courbevoie Cedex (FR)**

EP 0 028 964 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

# Procédé de préparation de difluorométhane sulfonates alcalins

La présente invention a pour objet un procédé de préparation de difluorométhane sulfonates alcalins ; elle concerne plus particulièrement la préparation de difluorométhane sulfonates alcalins à partir de chlorodifluorométhane.

On connaît dans l'art antérieur (J. Chem. Soc. 1960, 3058-62) un procédé pour préparer le difluorométhane sulfonate de sodium par réaction de chlorodifluorométhane avec du sulfite de sodium en solution aqueuse à 120 °C dans un autoclave. En opérant pendant 20 heures sous 16 bars de pression autogène, le rendement annoncé dans l'article précité est de 23 %. Les propres essais de la demanderesse, effectués dans les mêmes conditions, n'ont conduit qu'à une conversion de 4,4 % du chlorodifluorométhane avec une sélectivité en difluorométhane sulfonate de sodium de 10 %, ce qui conduit à un rendement global de 0,4 %.

La demanderesse a maintenant découvert un procédé palliant les inconvénients de l'art antérieur, c'est-à-dire, permettant d'obtenir des taux de conversion du chlorodifluorométhane et des sélectivités en difluorométhane sulfonate alcalin dignes d'intérêt au plan industriel.

La présente invention a donc pour objet un procédé de préparation de difluorométhane sulfonates alcalins par réaction d'un sulfite alcalin de formule $M_2SO_3$ dans laquelle M représente un métal alcalin de la colonne 1 de la classification périodique des éléments, avec une quantité au moins équimoléculaire de chlorodifluorométhane en milieu aqueux, en autoclave, sous pression autogène, caractérisé en ce que l'on met en œuvre la réaction en présence d'au moins une base alcaline forte de formule M'OH dans laquelle M' représente un métal alcalin de la colonne 1 de la classification périodique.

Selon un mode de réalisation préféré de l'invention, on met en œuvre la base alcaline forte correspondant au sulfite alcalin mis en réaction. Cela correspond au cas où M et M' représentent le même métal alcalin.

Le procédé selon l'invention est plus particulièrement adapté à la préparation du difluorométhane sulfonate de sodium et du difluorométhane sulfonate de potassium, car ce sont les deux composés qui ont le plus d'importance au plan industriel.

La réaction chimique intervenant dans le procédé selon l'invention peut s'écrire :

$$M_2SO_3 + Cl\ F_2\ CH \xrightarrow{M'OH} CH\ F_2SO_3M + MCl$$

où M et M' ont la signification précédente.

Selon un mode de réalisation préférentiel de l'invention, on utilise la base alcaline M'OH en quantité telle que le rapport molaire de la base alcaline M'OH au sulfite alcalin $M_2SO_3$ est compris entre 0,1 et 1. Encore plus préférentiellement, ce rapport est égal à environ 0,5.

On préfère opérer à une température comprise entre 100 °C et 200 °C. Encore plus préférentiellement, on opère à 150 °C environ.

Pour cette gamme de température, la pression qui s'établit dans l'autoclave est comprise entre 10 et 60 bars.

Selon un mode de réalisation particulier de l'invention, on met en œuvre la réaction en présence, en outre, d'au moins un agent de transfert de phase de préférence choisi parmi le groupe des sels d'ammonium quaternaires et de phosphonium quaternaires.

Cette adjonction, qui n'est pas impérative, permet d'augmenter la sélectivité en difluorométhane sulfonate alcalin.

Les sels d'ammonium et de phosphonium quaternaires utilisables dans le procédé de l'invention répondent aux formules générales :

$$R_1R_2R_3R_4N^+X^- \quad \text{et} \quad R_1R_2R_3R_4P^+X^-$$

dans lesquelles $R_1$, $R_2$, $R_3$ et $R_4$ identiques ou différents représentent chacun un élément choisi parmi le groupe comprenant les radicaux alkyle, aralkyle et aryle éventuellement substitués ayant de 1 à 24 atomes de carbone, et X représente un atome d'halogène ou $HSO_4^-$.

On préfère utiliser les sels d'ammonium ou de phosphonium quaternaires pour lesquels $R_1$, $R_2$, $R_3$ et $R_4$ identiques ou différents représentent chacun un élément choisi parmi le groupe comprenant les radicaux alkyle ayant de 1 à 12 atomes de carbone, les radicaux phényle et les radicaux benzyle et pour lesquels X représente le chlore ou le brome.

On peut citer comme exemples de tels composés :

$(C_6H_5CH_2)N^+(CH_3)_3Cl^-$, $(C_4H_9)_4N^+Cl^-$, $(C_8H_{17})_4N^+Br^-$,

$(C_6H_5CH_2)N^+(C_2H_5)_3Cl^-$, $(C_4H_9)_4N^+HSO_4^-$, $(C_{16}H_{33})P^+(C_4H_9)_3Br^-$,

$(C_{16}H_{33})P^+(C_6H_5)_3Cl^-$, $(C_6H_5CH_2)P^+(C_6H_5)_3Cl^-$, $(C_4H_9)_4P^+Br^-$.

Les sels d'ammonium ou des phosphonium quaternaires sont utilisés en quantité telle que le rapport molaire du sel d'ammonium ou de phosphonium quaternaire au sulfite alcalin est compris entre 0,02 et 0,1.

Encore plus préférentiellement, ce rapport est égal à environ 0,05.

Pour une bonne réalisation du procédé selon l'invention, on met en œuvre la réaction en présence d'une quantité d'eau supérieure ou égale à 200 ml par mole de sulfite alcalin engagée. De préférence, on utilise de 300 à 700 ml d'eau par mole de sulfite alcalin introduite dans le système réactionnel.

Les difluorométhanes sulfonates alcalins, en particulier le difluorométhane sulfonate de sodium, sont utiles, par exemple, pour la préparation du chlorure de difluorométhane sulfonyle qui est un intermédiaire de synthèse important, notamment pour la préparation de composés à activité pharmaceutique ou phytosanitaire.

D'autres caractéristiques et avantages de l'invention apparaitront plus clairement à la lecture des exemples qui suivent. Ces exemples ne sauraient en aucune façon être considérés comme une limitation de l'invention.

## Exemple 1

Un mélange de 53,1 g (0,6 mole) de chlorodifluorométhane, de 37,8 g (0,3 mole) de sulfite de sodium anhydre, de 12,0 g (0,3 mole) de soude en pastilles et de 100 ml d'eau est chauffé en autoclave pendant 20 heures à 120 °C sous une pression autogène de 16 bars. Après refroidissement, dégazage du chlorodifluorométhane non réagi, et ouverture de l'autoclave, on évapore le milieu réactionnel à sec. Le résidu solide obtenu est extrait à l'acétone. On récupère 38 g de produit solide insoluble dans l'acétone et l'évaporation à sec de la solution acétonique fournit 3,7 g de produit solide. Le produit insoluble dans l'acétone est constitué de sulfite de sodium non réagi, de chlorure de sodium (0,117 mole) de fluorure de sodium (0,091 mole) et d'un peu de difluorométhane sulfonate de sodium non extrait (0,009 mole). Le produit soluble dans l'acétone est constitué de difluorométhane sulfonate de sodium pratiquement pur (0,024 mole) légèrement souillé par du fluorure de sodium (0,000 4 mole).

Le rapport entre le nombre de moles de $CHF_2Cl$ réagies (indiqué par la quantité de $Cl^-$) le nombre de moles de $CHF_2Cl$ convertibles est de 39 %. La sélectivité en difluorométhane sulfonate de sodium (nombre de moles de $CHF_2SO_3Na$ formées par rapport au nombre de moles de $CHF_2Cl$ réagies) est de 28 %. Le rendement en difluorométhane sulfonate de sodium s'élève donc à 11 %.

## Exemple 2

Un mélange de 40,7 g (0,47 mole) de chlorodifluorométhane, de 18,9 g (0,15 mole) de sulfite de sodium anhydre, de 6,0 g (0,15 mole) de soude et de 50 ml d'eau, est chauffé en autoclave pendant 20 heures à 150 °C sous une pression autogène de 43 bars.

En utilisant le même traitement que dans l'exemple 1, on récupère 25,1 g de produit solide insoluble dans l'acétone et 5,3 g de produit solide soluble dans l'acétone.

La partie insoluble dans l'acétone est constituée de sulfite de sodium non réagi, de chlorure de sodium (0,156 mole), de fluorure de sodium (0,127 mole) et de difluorométhane sulfonate de sodium non extrait (0,011 mole).

La partie soluble dans l'acétone est constituée de difluorométhane sulfonate de sodium pur (0,035 mole). On constate qu'une quantité stœchiométrique de chlorodifluorométhane a réagi et que le rendement en difluorométhane sulfonate de sodium est de 29 %.

## Exemple 3

Un mélange de 156,0 g (1,8 mole) de chlorodifluorométhane, de 151,2 g (1,2 mole) de sulfite de sodium anhydre, de 24 g (0,6 mole) de soude et de 500 ml d'eau est traité dans les mêmes conditions que celles de l'exemple 2. On récupère ainsi 176 g de solide insoluble dans l'acétone et 94 g de solide soluble dans l'acétone. Le produit insoluble dans l'acétone est constitué de sulfite de sodium non réagi, de chlorure de sodium (1,122 mole) et de fluorure de sodium (0,867 mole).

Le produit soluble dans l'acétone est constitué de difluorométhane sulfonate de sodium pur en RMN$^{19}$F (0,612 mole).

Le rapport entre le nombre de moles de $CHF_2Cl$ réagies et le nombre de moles de $CHF_2Cl$ convertibles est de 93,5 % et la sélectivité en difluorométhane sulfonate de sodium est de 54,5 %, ce qui porte le rendement en $CHF_2SO_3Na$ à 51 %.

## Exemple comparatif

Un mélange de 0,45 mole de $CHF_2Cl$, de 0,3 mole de sulfite de sodium et de 125 ml d'eau est traité comme dans l'exemple 3.

Le rapport entre le nombre de moles de $CHF_2Cl$ réagies et le nombre de moles de $CHF_2Cl$ convertibles est de 70 % et la sélectivité en difluorométhanesulfonate de sodium est de 24 %, ce qui porte

le rendement en $CHF_2SO_3Na$ à 17 %.

Exemple 4

Un mélange de 79,0 g (0,91 mole) de chlorodifluorométhane, de 37,8 g (0,30 mole), de sulfite de sodium anhydre, de 6,0 g (0,15 mole) de soude, de 8,2 g (0,015 mole) de bromure de tétra-n-octylammonium et de 100 ml d'eau est traité dans les mêmes conditions que celles de l'exemple 3.

On obtient 43,9 g de solide insoluble dans l'acétone et 14,5 g de solide soluble dans l'acétone. La partie insoluble dans l'acétone est constituée de sulfite de sodium non réagi, de chlorure de sodium (0,208 mole), de fluorure de sodium (0,126 mole) et de difluorométhane sulfonate de sodium non extrait (0,027 mole).

La partie soluble dans l'acétone est constituée de difluorométhane sulfonate de sodium (0,088 mole) et de difluorométhane sulfonate d'ammonium (0,001 mole), que l'on peut distinguer du difluorométhane sulfonate de sodium en $RMN^{19}F$.

Le rapport entre le nombre de moles de $CHF_2Cl$ réagies et le nombre de moles de $CHF_2Cl$ convertibles est de 69 % et la sélectivité en difluorométhane sulfonate est de 56 % ce qui porte le rendement en $CHF_2SO_3^-$ à 39 %.

Exemple 5

Un mélange de 0,9 mole de chlorodifluorométhane, de 0,3 mole de sulfite de potassium, de 0,3 mole de potasse et de 100 ml d'eau est traité dans les mêmes conditions que celles de l'exemple 2. On obtient ainsi du difluorométhanesulfonate de potassium avec un rendement de 41 % par rapport au sulfite de potassium.

**Revendications**

1. Procédé de préparation de difluorométhane sulfonates alcalins par réaction d'un sulfite alcalin de formule $M_2SO_3$ dans laquelle M représente un métal alcalin de la colonne 1 de la classification périodique des éléments, avec une quantité au moins équimoléculaire de chlorodifluorométhane en milieu aqueux, en autoclave sous pression autogène, caractérisé en ce que l'on met en œuvre la réaction en présence d'au moins une base alcaline forte de formule M'OH dans laquelle M' représente un métal alcalin de la colonne 1 de la classification périodique des éléments.

2. Procédé selon la revendication 1 caractérisé en ce que M' et M représentent le même métal alcalin.

3. Procédé selon l'une quelconque des revendications 1 et 2, caractérisé en ce que M' et M identiques ou différents représentent le sodium ou le potassium.

4. Procédé selon la revendication 1 caractérisé en ce que l'on utilise la base alcaline en quantité telle que le rapport molaire de la base alcaline du sulfite alcalin est compris entre 0,1 et 1.

5. Procédé selon la revendication 4 caractérisé en ce que le rapport molaire de la base alcaline au sulfite alcalin est égal à environ 0,5.

6. Procédé selon l'une quelconque des revendications précédentes caractérisé en ce que l'on effectue la réaction à une température comprise entre 100 et 200 °C.

7. Procédé selon la revendication 6, caractérisé en ce que l'on effectue la réaction à environ 150 °C.

8. Procédé selon l'une quelconque des revendications précédentes caractérisé en ce que l'on effectue la réaction en présence, en outre, d'au moins un agent de transfert de phase.

9. Procédé selon la revendication 8 caractérisé en ce que l'agent de transfert de phase est choisi parmi le groupe comprenant les sels d'ammonium quaternaires et les sels phosphonium quaternaires.

10. Procédé selon la revendication 9 caractérisé en ce que les sels d'ammonium quaternaires et les sels de phosphonium quaternaires répondent aux formules :

$$R_1R_2R_3R_4N^+X^- \quad \text{et} \quad R_1R_2R_3R_4P^+X^-$$

dans lesquelles $R_1$, $R_2$, $R_3$ et $R_4$ identiques ou différents représentent chacun un élément choisi parmi le groupe comprenant les radicaux alkyle, aralkyle et aryle éventuellement substitués ayant de 1 à 24 atomes de carbone et X représente un atome d'halogène ou $HSO_4^-$.

11. Procédé selon la revendication 10 caractérisé en ce que le sel d'ammonium ou de phosphonium quaternaire est choisi parmi le groupe comprenant :

$(C_6H_5CH_2)N^+(CH_3)_3Cl^-$, $(C_4H_9)_4N^+Cl^-$, $(C_8H_{17})_4N^+Br^-$,

$(C_6H_5CH_2)N^+(C_2H_5)_3Cl^-$, $(C_4H_9)_4N^+HSO_4^-$, $(C_{16}H_{33})P^+(C_4H_9)_3Br^-$,

$(C_{16}H_{33})P^+(C_6H_5)_3Cl^-$, $(C_6H_5CH_2)P^+(C_6H_5)_3Cl^-$, $(C_4H_9)_4P^+Br^-$.

12. Procédé selon l'une quelconque des revendications 8 à 11 caractérisé en ce que le rapport molaire du sel d'alkylammonium au sulfite alcalin est compris entre 0,02 et 0,1.

13. Procédé selon la revendication 12 caractérisé en ce que le rapport molaire du sel d'alkylammonium au sulfite alcalin est égal à environ 0,05.

14. Procédé selon l'une quelconque des revendications précédentes caractérisé en ce que l'on effectue la réaction en présence d'au moins 200 ml d'eau par mole de sulfite alcalin engagée.

15. Procédé selon la revendication 14 caractérisé en ce que l'on effectue la réaction en présence de 300 ml à 700 ml d'eau par mole de sulfite alcalin engagée.

## Claims

1. Process for the preparation of alkali metal difluoromethanesulphonates by reacting an alkali metal sulphite of the formula $M_2SO_3$, in which M represents an alkali metal of column 1 of the periodic classification of the elements, with at least an equimolecular amount of chlorodifluoromethane in an aqueous medium, in an autoclave under autogenous pressure, characterised in that the reaction is carried out in the presence of at least one strong alkali metal base of the formula M'OH, in which M' represents an alkali metal of column 1 of the periodic classification of the elements.

2. Process according to Claim 1, characterised in that M' and M represent the same alkali metal.

3. Process according to either one of Claims 1 and 2, characterised in that M' and M, which are identical or different, represent sodium or potassium.

4. Process according to Claim 1, characterised in that the alkali metal base is used in an amount such that the molar ratio of the alkali metal base to the alkali metal sulphite is between 0.1 and 1.

5. Process according to Claim 4, characterised in that the molar ratio of the alkali metal base to the alkali metal sulphite is equal to about 0.5.

6. Process according to any one of the preceding claims, characterised in that the reaction is carried out at a temperature of between 100 and 200 °C.

7. Process according to Claim 6, characterised in that the reaction is carried out at about 150 °C.

8. Process according to any one of the preceding claims, characterised in that the reaction is carried out also in the presence of at least one phase transfer agent.

9. Process according to Claim 8, characterised in that the phase transfer agent is chosen from amongst the group comprising quaternary ammonium salts and quaternary phosphonium salts.

10. Process according to Claim 9, characterised in that the quaternary ammonium salts and the quaternary phosphonium salts correspond to the formulae :

$$R_1R_2R_3R_4N^+X^- \quad \text{and} \quad R_1R_2R_3R_4P^+X^-$$

in which $R_1$, $R_2$, $R_3$ and $R_4$, which are identical or different, each represent a radical chosen from amongst the group comprising optionally substituted alkyl, aralkyl and aryl radicals having from 1 to 24 carbon atoms, and X represents a halogen atom or $HSO_4^-$.

11. Process according to Claim 10, characterised in that the quaternary ammonium or phosphonium salt is chosen from amongst the group comprising :

$(C_6H_5CH_2)N^+(CH_3)_3Cl^-$, $(C_4H_9)_4N^+Cl^-$, $(C_8H_{17})_4N^+Br^-$,

$(C_6H_5CH_2)N^+(C_2H_5)_3Cl^-$, $(C_4H_9)_4N^+HSO_4^-$, $(C_{16}H_{33})P^+(C_4H_9)_3Br^-$,

$(C_{16}H_{33})P^+(C_6H_5)_3Cl^-$, $(C_6H_5CH_2)P^+(C_6H_5)_3Cl^-$, $(C_4H_9)_4P^+Br^-$.

12. Process according to any one of Claims 8 to 11, characterised in that the molar ratio of the alkylammonium salt to the alkali metal sulphite is between 0.02 and 0.1.

13. Process according to Claim 12, characterised in that the molar ratio of the alkylammonium salt to the alkali metal sulphite is equal to about 0.05.

14. Process according to any one of the preceding claims, characterised in that the reaction is carried out in the presence of at least 200 ml of water per mol of alkali metal sulphite used.

15. Process according to Claim 14, characterised in that the reaction is carried out in the presence of 300 ml to 700 ml of water per mol of alkali metal sulphite used.

## Ansprüche

1. Verfahren zur Herstellung von Alkalidifluormethansulfonaten durch Umsetzung eines Alkalisulfits der Formel $M_2SO_3$, in der M ein Alkalimetall der ersten Gruppe des periodischen Systems bedeutet, mit einer wenigstens äquimolaren Menge Chlordifluormethan in wässrigem Milieu in einem Autoklaven unter dem sich einstellenden Druck, dadurch gekennzeichnet, daß man die Reaktion in Gegenwart wenigstens

eines starken Alkalis der Formel M'OH durchführt, in der M' ein Alkalimetall der ersten Gruppe des periodischen Systems bedeutet.

2. Verfahren nach Anspruch 1, dadurch gekennzeichnet, daß M' und M das gleiche Alkalimetall bedeuten.

3. Verfahren nach Anspruch 1 oder 2, dadurch gekennzeichnet, daß M' und M gleich oder verschieden sind und Natrium oder Kalium bedeuten.

4. Verfahren nach Anspruch 1, dadurch gekennzeichnet, daß man das Alkali in einer solchen Menge verwendet, daß das Molverhältnis des Alkalis zum Alkalisulfit zwischen 0,1 und 1 liegt.

5. Verfahren nach Anspruch 4, dadurch gekennzeichnet, daß das Molverhältnis des Alkalis zum Alkalisulfit etwa 0,5 beträgt.

6. Verfahren nach einem der vorangehenden Ansprüche, dadurch gekennzeichnet, daß man die Umsetzung bei einer Temperatur zwischen 100 und 200 °C durchführt.

7. Verfahren nach Anspruch 6, dadurch gekennzeichnet, daß man die Reaktion bei etwa 150 °C durchführt.

8. Verfahren nach einem der vorangehenden Ansprüche, dadurch gekennzeichnet, daß man die Reaktion zusätzlich in Gegenwart wenigstens eines Phasenübertragungsmittels durchführt.

9. Verfahren nach Anspruch 8, dadurch gekennzeichnet, daß das Phasenübertragungsmittel aus der Gruppe der quaternären Ammonium- oder Phosphoniumsalze gewählt ist.

10. Verfahren nach Anspruch 1, dadurch gekennzeichnet, daß die quaternären Ammonium- und Phosphoniumsalze die Formeln :

$$R_1R_2R_3R_4N^+X^- \quad \text{und} \quad R_1R_2R_3R_4P^+X^-$$

haben, in denen $R_1$, $R_2$, $R_3$ und $R_4$ gleich oder verschieden sind und einen gegebenenfalls substituierten Alkyl-, Aralkyl- oder Arylrest mit 1 bis 24 Kohlenstoffatomen bedeuten und X ein Halogenatom oder $HSO_4^-$ ist.

11. Verfahren nach Anspruch 10, dadurch gekennzeichnet, daß das quaternäre Ammonium- oder Phosphoniumsalz aus der folgenden Verbindungsgruppe gewählt ist :

$(C_6H_5CH_2)N^+(CH_3)_3Cl^-$, $(C_4H_9)_4N^+Cl^-$, $(C_8H_{17})_4N^+Br^-$,

$(C_5H_5CH_2)N^+(C_2H_5)_3Cl^-$, $(C_4H_9)_4N^+HSO_4^-$, $(C_{16}H_{33})P^+(C_4H_9)_3Br^-$,

$(C_{16}H_{33})P^+(C_6H_5)_3Cl^-$, $(C_6H_5CH_2)P^+(C_6H_5)_3Cl^-$, $(C_4H_9)_4P^+Br^-$.

12. Verfahren nach einem der Ansprüche 8 bis 11, dadurch gekennzeichnet, daß das Molverhältnis des Alkylammoniumsalzes zum Alkalisulfit zwischen 0,02 und 0,1 liegt.

13. Verfahren nach Anspruch 12, dadurch gekennzeichnet, daß das Molverhältnis des Alkylammoniumsalzes zum Alkalisulfit etwa 0,05 beträgt.

14. Verfahren nach einem der vorangehenden Ansprüche, dadurch gekennzeichnet, daß man die Reaktion in Gegenwart von wenigstens 200 ml Wasser pro Mol des eingesetzten Alkalisulfits durchführt.

15. Verfahren nach Anspruch 14, dadurch gekennzeichnet, daß man die Reaktion in Gegenwart von 300 bis 700 ml Wasser pro Mol des eingesetzten Alkalisulfits durchführt.